Europäisches Patentamt

European Patent Office

Office européen des brevets

(11) Veröffentlichungsnummer : **0 075 742**
**B1**

(12) # EUROPÄISCHE PATENTSCHRIFT

(45) Veröffentlichungstag der Patentschrift :
07.11.84

(21) Anmeldenummer : 82108033.0

(22) Anmeldetag : 01.09.82

(51) Int. Cl.³ : **C 07 C 19/045**, C 07 C 17/02,
C 07 C 17/38

(54) **Verfahren zur Herstellung von 1,2-Dichlorethan.**

(30) Priorität : 21.09.81 DE 3137513
09.07.82 DE 3225732

(43) Veröffentlichungstag der Anmeldung :
06.04.83 Patentblatt 83/14

(45) Bekanntmachung des Hinweises auf die Patenterteilung : 07.11.84 Patentblatt 84/45

(84) Benannte Vertragsstaaten :
BE CH DE FR GB IT LI NL SE

(56) Entgegenhaltungen :
EP-A- 0 001 417
EP-A- 0 026 349
DE-A- 1 543 108
DE-A- 2 224 253
DE-A- 2 427 045
GB-A- 1 231 127

(73) Patentinhaber : HOECHST AKTIENGESELLSCHAFT
Postfach 80 03 20
D-6230 Frankfurt am Main 80 (DE)

(72) Erfinder : Hundeck, Joachim, Dr.
Argelanderstrasse 135
D-5300 Bonn 1 (DE)
Erfinder : Scholz, Harald, Dr.
AM Beissel 8
D-5042 Erftstadt 12 (DE)
Erfinder : Hennen, Hans
Kendenicher Strasse 86
D-5030 Hürth (DE)
Erfinder : Kuxdorf, Bernhard, Dipl.-Ing.
Von Westerburgstrasse 12
D-5040 Brühl (DE)
Erfinder : Püsche, Herbert, Dipl.-Ing.
Elsterweg 30
D-5042 Erftstadt (DE)
Erfinder : Vomberg, Heinz, Dipl.-Ing.
Peter-May-Strasse 17
D-5042 Erftstadt (DE)
Erfinder : Link, Gerhard, Dipl.-Ing.
Aubachstrasse 55
D-6500 Mainz (DE)

**Beschreibung**

Die Erfindung betrifft ein Verfahren zur Herstellung und Reinigung von 1,2-Dichlorethan durch Umsetzung von Ethylen und Chlor in flüssigem 1,2-Dichlorethan in Gegenwart eines hierfür üblichen Katalysators, wobei die bei der Reaktion entstehende Reaktionswärme nutzbringend verwendet und die unerwünschte Bildung höher chlorierter Produkte, wie Tri-, Tetra- und Pentachlorethan, im Reaktor weitgehend vermieden sowie die Kumulierung derartiger Produkte in der Reaktionszone verhindert wird.

Die Chlorierung von Olefinen mittels Chlor ist bekanntlich eine exotherme Reaktion. Im Falle der Chlorierung von Ethylen mit Chlor beträgt die Wärmetönung 2.200 kJ pro Kilogramm 1,2-Dichlorethan. Bei der Produktion einer Tonne 1,2-Dichlorethan fällt somit eine Wärmemenge an, die ausreicht, um etwa 1 Tonne Dampf zu erzeugen. Bei den bekannten Verfahren zur Herstellung von Dichloretan wurde die Reaktionswärme entweder durch Kühlung des Reaktors abgeführt oder sie wurde teilweise zur unmittelbaren Verdampfung und Abtreibung des bei der Reaktion entstandenen Dichlorethans aus dem Reaktionsgemisch bzw. dem Reaktor und in einzelnen Fällen auch bereits zur auschließlichen Rektifizierung von nach anderer Verfahrensweise hergestelltem 1,2-Dichlorethan mehr oder weniger vollständig genutzt.

Eine kombinierte Abführung der Reaktionswärme bei der Chlorierung von Ethylen mit Chlor wird ferner beispielsweise bei dem in der DE-PS 15 43 108 beschriebenen Verfahren praktiziert. Hierbei ist der zur Durchführung der Reaktion vorgesehene Eisenreaktor mit einer Kühlvorrichtung versehen, welche mit Kühlwasser beaufschlagt ist, um bei der Reaktion entstehende Wärme abzuführen und die für die Umsetzung vorbestimmte Reaktionstemperatur von 50-70 °C einhalten zu können. Die Einhaltung der unterhalb des Siedepunktes von 1,2-Dichlorethan liegenden Reaktionstemperatur wird während der Reaktions so geregelt, daß das gebildete 1,2-Dichlorethan in dampfförmigem Zustand kontinuierlich aus dem Reaktionsraum entfernt wird. Bei dieser Verfahrensweise findet zwar keine Kumulierung höher chlorierter Produkte im Reaktor statt, doch werden dabei 3,3 % Trichlorethan gebildet und die Reaktionswärme kann nicht genutzt werden, weil das kondensierte 1,2-Dichlorethan noch von dem erwähnten Nebenprodukt befreit werden muß.

Ein im Prinzip mit dem Verfahren der DE-PS 15 43 108 übereinstimmendes Verfahren wird in der DE-OS 29 35 884 offenbart, wobei die charakteristischen Merkmale letztgenannten Verfahrens darin bestehen, daß die Reaktorfüllung während der Reaktion über eine mit dem Reaktor in Verbindung stehenden Ringleitung umgewälzt wird und die am Kopf des Reaktors abziehenden dampfförmigen Produkte in einer Rektifizierkolonne unter Gewinnung von 1,2-Dichlorethan getrennt werden. Höher siedende Nebenprodukte werden dabei nachteiligerweise vom Sumpf der Rektifizierkolonne in den Reaktor zurückgeleitet, wodurch in diesem Falle eine separate Aufarbeitung diskontinuierlich entnommener Mengen Reaktionssumpfprodukt erforderlich ist und die Reaktionswärme nur teilweise genutzt werden kann.

Schließlich besteht eine weitere Verfahrensweise zur Herstellung von Ethylenchlorid gemäß DE-OS 24 27 045 darin, daß man

a) Ethylen und Chlor in eine unter erhöhtem Druck stehende Reaktionszone, welche ein umlaufendes flüssiges Medium mit einem Gehalt an chlorierten Kohlenwasserstoffen mit zwei Kohlenstoffatomen oder Gemischen dieser chlorierten Kohlenwasserstoffe enthält, das bei einer Temperatur unterhalb der Verdampfungstemperatur des Mediums bei dem in der Reaktionszone herrschenden Druck gehalten wird, einleitet, wobei rohes flüssiges Ethylendichlorid gebildet wird ;

b) das rohe flüssige Ethylendichlorid mit dem umlaufenden Medium zu einer unter niedrigerem Druck stehenden Zone führt, wobei man Druck und Temperatur dieser Zone auf Werten hält, bei denen das unreine Ethylendichlorid durch die bei der Umsetzung von Chlor und Ethylen freiwerdende Reaktionswärme verdampft und

c) das dampfförmige unreine Ethylendichlorid in eine Rektifikationszone einleitet und mit Hilfe der bei der Rekation von Chlor und Ethylen freiwerdenden Reaktionswärme rektifiziert, wobei das gereinigte Ethylendichlorid aus der Rektifikationszone abgezogen wird, während das Sumpfprodukt der Rektifizierkolonne in die Zone niedrigeren Drucks zurückgeführt und mit dem umlaufenden Medium vereinigt wird.

Die Rückführung des Sumpfproduktes in den Reaktor ist insofern nachteilig, als das umlaufende flüssige Medium mit höher siedenden Chlorierungsprodukten angereichert wird, welche aus letzterem entfernt werden müssen. Gemäß Beispiel 4 der DE-OS 24 27 045 beträgt der Anteil an 1,1,2-Trichlorethan im umlaufenden Medium etwa 60 %. Dies bedeutet, daß die bekannte Chlorierungsreaktion unter Bildung erheblicher Anteile an unerwünschten Nebenprodukten verläuft.

Es bestand somit die Aufgabe, die bekannten Verfahrensweisen in dem Maße zu verbessern, daß die Bildung von Nebenprodukten weitgehend vermieden und die bei der Reaktion anfallende Reaktionswärme optimal genutzt wird. Diese Aufgabe wird primär dadurch gelöst, daß aus dem Reaktionsgemisch bei einer vorgegebenen Verweilzeit der Gemischkomponenten stets der produzierte Anteilan Rohdichlorethan dampfförmig abgezogen und in einer separaten Rektifizierkolonne gereinigt wird. Eventuell als Sumpfprodukt anfallende höher chlorierte Nebenprodukte werden aus der Rektifizierkolonne ohne Rückführung in die Reaktionszone der Ausgangsprodukte abgezogen und einer anderweitigen Verwendung zugeführt. Die Rektifikation des Rohdichlorethans kann mit Hilfe der bei der Umsetzung von Ethylen mit Chlor freiwerdenden Wärmeenergie erfolgen, wobei letztere nur zum Teil verbraucht wird, während die Restwärme zur weiteren Verwendung in von dem vorliegenden Verfahren unabhängiger Weise, z. B. zur Dampferzeugung, genutzt werden kann.

Gegenstand der Erfindung ist somit ein Verfahren zur Herstellung von 1,2-Dichlorethan durch Umsetzung von Ethylen und Chlor in einer Reaktionszone, welche ein umlaufendes flüssiges Medium mit einem Gehalt an chlorierten Kohlenwasserstoffen mit zwei Kohlenstoffatomen enthält, bei einer Temperatur unterhalb der Verdampfungstemperatur des Mediums bei dem in der Reaktionszone herrschenden Druck und in Gegenwart eines üblichen Katalysators zur Chlorübertragung und gegebenenfalls eines Inhibitors zur Verringerung der Nebenproduktbildung, unter Bildung von rohem 1,2-Dichlorethan, welches aus der Reaktionszone abgezogen und in einer anschließenden separaten Fraktionierkolonne gereinigt wird, welches dadurch gekennzeichnet ist, daß man

a) etwa äquimolare Mengen von Ethylen und Chlor in das umlaufende flüssige Medium einleitet und nach intensiver Durchmischung in einer Mischzone das Gemisch in einer Reaktionszone bei einer Temperatur von etwa 75-200 °C und einem Druck von etwa 1-15 bar zur Reaktion bringt, wobei die mittlere Verweilzeit des Reaktionsgemisches in der Misch- und Reaktionszone etwa 1-15 Stunden beträgt ;

b) aus der Reaktionszone einen Teil des flüssigen Reaktionsgemisches abzieht und letzteren in zwei Teilströme aufteilt, wobei ein Teilstrom zur Abgabe von Wärmeenergie einen Wärmeaustauscher passiert und danach mit verminderter Temperatur in die Misch- und Reaktionszone zurückfließt, während der zweite Teilstrom einem Entspannungsgefäß zugeführt wird, in welchem eine adäquate Menge des in der Reaktionszone gebildeten Reaktionsproduktes sowie gegebenenfalls ein Anteil nach anderer Verfahrensweise hergestelltes und der Reaktionszone zugeführtes 1,2-Dichlorethan aus dem zweiten Teilstrom verdampft, wobei die Dämpfe in eine Fraktionierkolonne eingeleitet werden, während der nicht verdampfte, flüssige Anteil des zweiten Teilstromes in die Misch- und Reaktionszone des umlaufenden flüssigen Mediums zurückkehrt, und

c) aus den in die Fraktionierkolonne eingeleiteten Dämpfen das 1,2-Dichlorethan destillativ unter Verwendung eines Teiles der im Wärmeaustauscher übertragenen Wärmeenergie abtrennt und letzteres über Kopf der Kolonne abzieht, wobei im Sumpf der Kolonne höher chlorierte Produkte anfallen, die abgezogen und separat aufgearbeitet werden.

Einer bevorzugten Ausführungsform der Erfindung entsprechend, erfolgt die Umsetzung des Ethylens mit Chlor bei einer Temperatur von 95-160 °C, einem Druck von 1-15 bar und unter Einhaltung einer mittleren Verweilzeit von 2 bis 10 Stunden. Das umlaufende flüssige Medium besteht vorteilhafterweise überwiegend aus 1,2-Dichlorethan, wobei letzteres mit geringen Mengen von bei der Reaktion als Nebenprodukte entstehenden höheren Chlorierungsprodukten des Ethylens verunreinigt sein kann. Der zum Wärmeaustausch abgezogene Teilstrom des flüssigen Reaktionsgemisches kehrt nach Passieren des Wärmetauschers mit einer um etwa 5 bis 50 °C verminderten Temperatur in die Misch- und Reaktionszone zurück.

Sofern die Ausgangsprodukte mit inerten Gasen verdünnt sind, werden diese aus dem Reaktionsgemisch zusammen mit leichtsiedenden Chlorkohlenwasserstoffen, wie Ethylchlorid, derart entfernt, daß man sie aus dem oberen Teil der Reaktionszone abzieht und in einem nachgeschalteten Kühler kühlt, wobei mit den inerten Gasen entweichendes Dichlorethan kondensiert und in die Reaktionszone zurückgeleitet oder einem anderen Verwendungszweck zugeführt wird.

Schließlich kann in das erfindungsgemäße Verfahren auch die Reinigung von rohem 1,2-Dichlorethan, welches nach anderen Verfahrensweisen als das der Erfindung hergestellt wurde, einbezogen werden. Zu diesem Zweck leitet man das rohe Dichlorethan entweder in den dem Reaktor nachgeschalteten Kühler ein und führt es über die Abflußleitung des Kühlers der Reaktionszone zu oder man bringt es in einen der beiden Teilströme nach Wärmeaustausch bzw. Produktverdampfung vor deren Rückkehr in die Reaktionszone ein. Die aus der Reaktionszone abgezogene Menge umlaufenden Reaktionsgemisches entspricht etwa dem 3- bis 30-fachen des Reaktorvolumens. Die anfallende nutzbare Wärmeenergie kann zur Rektifizierung des gebildeten sowie einer zusätzlichen Menge nach anderer Verfahrensweise hergestellten 1,2-Dichlorethans herangezogen werden, sie kann aber auch ganz oder teilweise außerhalb des Verfahrens für Heizzwecke oder zur Dampferzeugung verwendet werden.

Im einzelnen ist zu dem Verfahren der Erfindung noch folgendes zu bemerken :

Die Reaktionspartner Ethylen und Chlor können durch inerte Gase verdünnt sein. Chlor kann in flüssiger Form oder als Gas in die Mischzone eingebracht werden, jedoch ist es zweckmäßig, flüssiges Chlor vor Eintritt in den Reaktor mit Hilfe eines Teiles der Reaktionsenthalpie in einem Wärmeaustauscher zu verdampfen. Als Katalysator empfiehlt sich die Verwendung von Eisen-III-chlorid und als Inhibitor zur Vermeidung der Nebenproduktbildung vorzugsweise Sauerstoff.

Der Umlauf des flüssigen Mediums im Reaktor kann beispielsweise mittels Pumpe und/oder nach dem Thermosiphon- bzw. Mammutpumpenprinzip bewirkt werden. Die Umlaufgeschwindigkeit des flüssigen Mediums soll in der Mischzone nicht kleiner als 0,1 m/sec sein. Der Umlauf zum Wärmeaustausch und zur Produktverdampfung kann ebenfalls mittels Pumpe und/ oder nach dem Thermosiphonprinzip erfolgen, es ist sogar möglich, beide Vorgänge hintereinandergeschaltet in einem einzigen Kreislauf flüssigen Mediums ablaufen zu lassen.

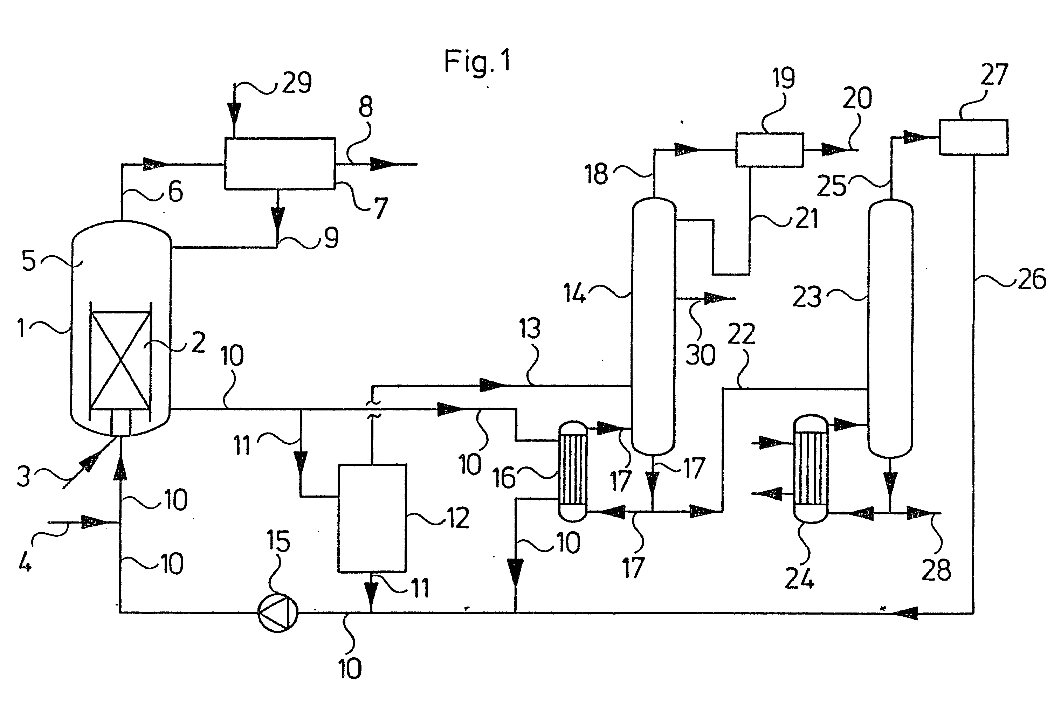

Eine beispielhafte Ausführungsform des Verfahrens der Erfindung wird im folgenden in Verbindung mit dem Fließschema gemäß Figur 1 näher erläutert, wobei das Verfahren auf diese Ausführungsform nicht beschränkt ist.

Ein zylindrischer Reaktor 1, der mit einem unten und oben offenen Innenzylinder 2 als Mischzone versehen ist, wobei der Innenzylinder 2 Füllkörper oder Einbauten enthält, wird zunächst mit flüssigem 1,2-Dichlorethan beschickt und letzteres durch Einleiten von Ethylen in den Reaktor 1 über die Leitung 3 sowie Chlorgas über die Leitung 4 nach dem Mammutpumpenprinzip in Zirkulation versetzt. Nach Eintreten der Reaktion des Ethylens mit dem Chlorgas, die in der Mischzone beginnt und in der Reaktionszone 5 vervollständigt wird, entsteht ein zusätzlicher Auftrieb im Innenzylinder 2, sofern in den Ausgangsprodukten inerte Gase enthalten sind, sowie durch die Temperaturdifferenz infolge der freiwerdenden Reaktionswärme. Die Temperatur im Reaktor 1 liegt etwas niedriger, als die Siedetemperatur des 1,2-Dichlorethans bei dem im Reaktor herrschenden Druck. Eventuell im Reaktor 1 vorhandene inerte Gase, werden über die Leitung 6 abgezogen und im Kühler 7 zur Kondensation von mitgeführten 1,2-Dichlorethandämpfen gekühlt. Die nicht kondensierten Gase werden über die Leitung 8 abgeblasen, während das Kondensat über die Leitung 9 in den Reaktor 1 zurückfließt. Durch Regulierung des Inertgasstromes kann im Reaktor 1 der gewünschte Druck eingestellt werden. Als Zuführungsleitung für Rohdichlorethan anderer Herkunft ist die Leitung 29 vorgesehen.

Zur Gewinnung des im Reaktor 1 produzierten 1,2-Dichlorethans wird aus dem Reaktor ein Flüssigkeitsstrom des Reaktionsgemisches über die Kreislaufleitung 10 entnommen, in zwei Teilströme aufgeteilt, wobei ein Teilstrom seinen Wärmeinhalt an den Wärmeaustauscher 16 abgibt und im Kreislauf über die Kreislaufleitung 10 in den Reaktor 1 zurückkehrt, während der zweite Teilstrom über die Leitung 11 dem Entspannungsgefäß 12 zufließt. Die Menge des den Reaktor 1 verlassenden Flüssigkeitsstromes beträgt etwa das Fünfzehnfache des Gesamtvolumens der Reaktorfüllung.

Im Entspannungsgefäß 12 wird aus dem eingeleiteten Flüssigkeitsstrom eine adäquate Menge des in der Reaktionszone gebildeten Reaktionsproduktes verdampft, wobei die Dämpfe über die Leitung 13 in die Fraktionierkolonne 14 geleitet werden, während der nichtverdampfte, flüssige Anteil vom Entspannungsgefäß 12 abgezogen und über die Leitungen 11 und 10 in den Reaktor 1 mit Hilfe der Pumpe 15 zurückgeführt wird. In der Kolonne 14 werden die eingeleiteten Dämpfe fraktioniert destilliert. Die hierfür erforderliche Wärmeenergie wird dem Wärmeaustauscher 16 entnommen, indem das Sumpfprodukt der Kolonne 14 über die Kreislaufleitung 17 dem Wärmeaustauscher 16 zugeführt wird. Reines 1,2-Dichlorethan wird am Kopf der Kolonne 14 über die Leitung 18 abgezogen, im Kühler 19 kondensiert und vom Kühler 19 über die Leitung 20 abgeführt. Ein Teil des Kondensates dient als Rückfluß für die Kolonne 14 und wird letzterer über die Leitung 21 zugeführt. Sofern das in der Kolonne 14 destillierte Gemisch neben 1,2-Dichlorethan noch leichter siedende Komponenten enthält, werden letztere über die Leitung 20 abgezogen und das 1,2-Dichlorethan über die Leitung 30 entnommen.

Eine verstärkte Reinigung des aus überwiegend höherchlorierten Produkten des Ethylens sowie geringen Restmengen an 1,2-Dichlorethan bestehenden Sumpfproduktes der Kolonne 14 kann gegebenenfalls dadurch bewirkt werden, daß man das Sumpfprodukt von der Kolonne 14 über die Leitungen 17 und 22 in die Kolonne 23 einbringt und im Vakuum destilliert.

Auch in diesem Falle kann die für die Destillation erforderliche Wärmeenergie aus einem Wärmeaustauscher 24 bezogen werden, dessen Wärmeinhalt aus der Reaktionsenthalpie gedeckt werden kann. Das Kopfprodukt der Kolonne 23 wird über die Leitungen 25, 26 und 10 nach vorheriger Verflüssigung im Kondensator 27 dem Reaktor 1 zugeführt. Das Sumpfprodukt der Kolonne 23 wird über die Leitung 28 entnommen.

Das vorbeschriebene Verfahren der Erfindung zeichnet sich gegenüber den bekannten Verfahren durch verschiedene Verfahrensvorteile aus. Einer dieser Vorteile resultiert aus der kontinuierlichen Entnahme eines Teiles des flüssigen Reaktorinhaltes und dessen Aufteilung in zwei Teilströme, wovon ein Teilstrom die laufende Rückgewinnung und Übertragung der Reaktionswärme ermöglicht, während die partielle Verdampfung des zweiten Teilstromes und die ausschließliche Aufarbeitung des Dampfanteiles dazu führt, daß nur katalysatorfreies Rohdichlorethan in die Fraktionierkolonne gelangt. Mit vorgenannten Dämpfen werden aber auch die höherchlorierten Nebenprodukte der Fraktionierkolonne zugeführt, d. h. daß der flüssige Anteil des zweiten Teilstromes um den Anteil von Nebenprodukten vermindert, der bei der Reaktion entstanden ist, in den Reaktor zurückfließt, wodurch eine Kumulierung von Nebenprodukten im Reaktorsumpf vermieden wird. Letztere bewirkt nämlich bei den bekannten Verfahren eine Anreicherung höherchlorierter Nebenprodukte und zwingt zur Abtrennung der Nebenprodukte aus dem Reaktorsumpf. Hierbei eintretende Katalysatorverluste müssen ergänzt werden, wodurch die Wirtschaftlichkeit dieser Verfahren beeinträchtigt wird. Durch den erfindungsgemäßen Verfahrensablauf wird die mittlere Verweilzeit aller an der Reaktion beteiligten Stoffe unter den gegebenen Reaktionsbedingungen im Reaktor so weit vermindert, daß unerwünschte, zur Nebenproduktbildung führende Nebenreaktionen weitgehend vermieden werden. Schließlich ermöglicht das Verfahren der Erfindung auch den Einsatz von mit Inertgasen verunreinigten Ausgangsprodukten, die bekannterweise bei der Reinigung des Dichlorethans in der Fraktionierkolonne Schwierigkeiten bereiten. Erfindungsgemäß werden diese Inertgase bereits aus dem Reaktor über die Inertgasleitung abgezogen, so daß sie den weiteren Aufarbeitungsprozeß des Reaktionsgemisches nicht stören können. Für die Durchführung des erfindungsgemäßen Verfahrens ist keine besondere, aufwendige Vorrichtung erforderlich. So ist es möglich, bereits vorhandene herkömmliche Einrichtungen, wie sie zur Additionschlorierung von Ethylen bisher benutzt wurden, mit geringen Mitteln umzurüsten, was sich zusätzlich vorteilhaft auf die Wirtschaftlichkeit des Verfahrens auswirkt.

Wie bereits ausgeführt, kann in das erfindungsgemäße Verfahren auch die Reinigung von rohem 1,2-Dichlorethan, welches nach anderen Verfahrensweisen als das der Erfindung hergestellt wurde, einbezogen werden.

Die Reinigung von nach anderer Verfahrensweise hergestelltem rohem 1,2-Dichlorethan — im folgenden Fremd-EDC genannt — im Zuge der Herstellung von 1,2-Dichlorethan durch Chlorierung von Ethylen mit Chlor wird bereits in der DE-OS 24 27 045 vorgeschlagen. Danach wir der Fremd-EDC-Strom, der beispielsweise aus dem Oxychlorierungsprozess entstammen kann, vorzugsweise als Flüssigkeit mit einer Temperatur von etwa 85 °C bis 130 °C unter dem herrschenden Druck in den Chlorierungsreaktor eingeleitet, in welchem er einen Teil des umlaufenden Mediums bilden kann bzw. durch die bei der Reaktion von Ethylen mit Chlor gebildete Reaktionswärme verdampft, wobei die Dämpfe einer nachfolgenden Fraktionierkolonne zuströmen. Hochsiedende Verunreinigungen, sowohl aus der direkten Chlorierungsreaktion als auch aus dem Fremd-EDC-Strom, die sich im Reaktionsmedium ansammeln, werden von Zeit zu Zeit aus dem Reaktor abgezogen.

Mit steigender Zuführung von Fremd-EDC in den Chlorierungsreaktor ist verständlicherweise ein Druckanstieg in Kolonne und Reaktor verbunden. Im Falle des Verfahrens der DE-OS 24 27 045 wirkt sich der Druckanstieg auf die Verdampfungsrate des 1,2-Dichlorethans im Reaktor und dessen Abtreibung aus dem Reaktor negativ aus. Bei der vorbeschriebenen erfindungsgemäßen Verfahrensweise sinkt mit steigender Zuführung von Fremd-EDC in den Chlorierungsreaktor die Druckdifferenz zwischen Kolonne und Reaktor. Bei gegebenem Druck des zugeführten Chlorgases kann der Reaktordruck nicht beliebig erhöht werden, so daß die Verdampfungsrate aus dem Reaktionsmedium bei weiterer Steigerung der Fremd-EDC-Zufuhr abfallen würde.

Vorerwähnter Nachteil kann überwunden werden, indem in Durchführung des erfindungsgemäßen Verfahrens dafür gesorgt wird, daß zwischen dem Druck im Reaktor und dem Druck in der Fraktionierkolonne ein ausreichendes Druckgefälle besteht, wodurch die Möglichkeit gegeben ist, mehr Fremd-EDC aus dem Reaktionsgemisch heraus zu verdampfen, auch wenn die vorgegebenen Druckverhältnisse und Apparatedimensionen keine Steigerung mehr zulassen würden.

Die Modifizierung des erfindungsgemäßen Verfahrens zum Zwecke der zusätzlichen Reinigung von Fremd-EDC ist dadurch gekennzeichnet, daß man an die zur Abtrennung des 1,2-Dichlorethans aus dem Reaktionsgemisch bestimmte Fraktionierkolonne ein Vakuum anlegt derart, daß das Druckverhältnis zwischen Reaktordruck und dem Druck in der Fraktionierkolonne etwa 2 bis 10 zu 1 beträgt.

Nach einer bevorzugten Ausführungsform der modifizierten Arbeitsweise beträgt der Druck im Reaktor etwa 1 bis 5 bar und der Druck in der Fraktionierkolonne etwa 0,9 bis 0,2 bar, insbesondere 0,7-0,5 bar.

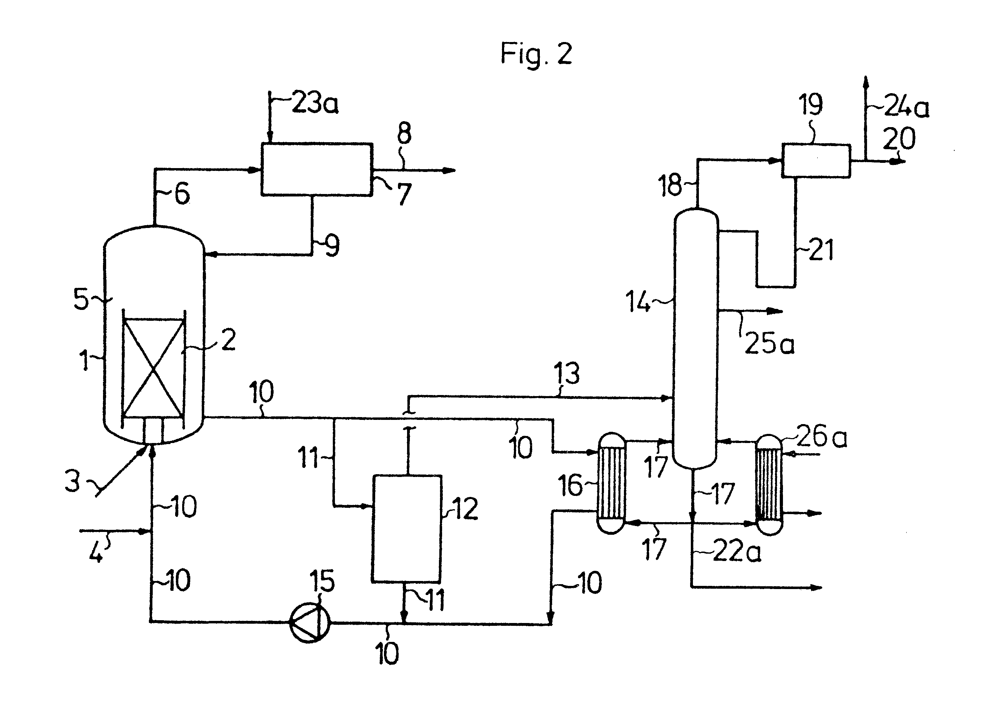

Eine beispielhafte Ausführungsform des modifizierten erfindungsgemäßen Verfahrens wird im folgenden in Verbindung mit dem Fließschema gemäß Figur 2 näher erläutert, wobei das Verfahren auf diese Ausführungsform nicht beschränkt ist.

Ein zylindrischer Reaktor 1, der mit einem unten und oben offenen Innenzylinder 2 als Mischzone versehen ist, wobei der Innenzylinder 2 Füllkörper oder Einbauten enthält, wird zunächst mit flüssigem 1,2-Dichlorethan beschickt und letzteres durch Einleiten von Ethylen in den Reaktor 1 über die Leitung 3 sowie Chlorgas über die Leitung 4 nach dem Mammutpumpenprinzip in Zirkulation versetzt. Nach Eintreten der Reaktion des Ethylens mit dem Chlorgas, die in der Mischzone beginnt und in der Reaktionszone 5 vervollständigt wird, entsteht ein zusätzlicher Auftrieb im Innenzylinder 2, sofern in den Ausgangsprodukten inerte Gase enthalten sind, sowie durch die Temperaturdifferenz infolge der freiwerdenden Reaktionswärme. Die Temperatur im Reaktor 1 liegt etwas niedriger als die Siedetemperatur des 1,2-Dichlorethans bei dem im Reaktor herrschenden Druck. Eventuell im Reaktor 1 vorhandene inerte Gase werden über die Leitung 6 abgezogen und im Kühler 7 zur Kondensation von mitgeführten 1,2-Dichlorethandämpfen gekühlt. Die nicht kondensierten Gase werden über die Leitung 8 abgeblasen, während das Kondensat über die Leitung 9 in den Reaktor 1 zurückfließt. Durch Regulierung des Inertgasstromes kann im Reaktor 1 der gewünschte Druck eingestellt werden. Als Zuführungsleitung für Rohdichlorethan anderer Herkunft ist die Leitung 23a vorgesehen.

Zur Gewinnung des im Reaktor 1 produzierten 1,2-Dichlorethans wird aus dem Reaktor ein Flüssigkeitsstrom des Reaktionsgemisches über die Kreislaufleitung 10 entnommen, in zwei Teilströme aufgeteilt, wobei ein Teilstrom seinen Wärmeinhalt an den Wärmeaustauscher 16 abgibt und im Kreislauf über die Kreislaufleitung 10 in den Reaktor 1 zurückkehrt, während der zweite Teilstrom über die Leitung 11 dem Entspannungsgefäß 12 zufließt. Die Menge des den Reaktor 1 verlassenden Flüssigkeitsstromes beträgt etwa das Fünfzehnfache des Gesamtvolumens der Reaktorfüllung.

Im Entspannungsgefäß 12 wird aus dem eingeleiteten Flüssigkeitsstrom eine adäquate Menge des in der Reaktionszone gebildeten Reaktionsproduktes und das zugeführte Fremd-EDC verdampft, wobei die Dämpfe über die Leitung 13 in die Fraktionierkolonne 14 geleitet werden, während der nichtverdampfte, flüssige Anteil vom Entspannungsgefäß 12 abgezogen und über die Leitungen 11 und 10 in den Reaktor 1 mit Hilfe der Pumpe 15 zurückgeführt wird. In der Kolonne 14 werden die eingeleiteten Dämpfe bei vermindertem Druck fraktioniert destilliert, wobei die Druckminderung in der Kolonne 14 durch Anlegen einer Vakuumpumpe an die Leitung 24a bewirkt wird. Die zur Destillation erforderliche Wärmeenergie wird dem Wärmeaustauscher 16 entnommen, indem das Sumpfprodukt der Kolonne 14 über die Kreislaufleitung 17 dem Wärmeaustauscher 16 zugeführt wird. Reines 1,2-Dichlorethan wird am Kopf der Kolonne 14 über die Leitung 18 abgezogen, im Kühler 19 kondensiert und vom Kühler 19 über die Leitung 20 abgeführt. Ein Teil des Kondensates dient als Rückfluß für die Kolonne 14 und wird letzterer über die Leitung 21 zugeführt. Für den Fall, daß die gesamte Reaktionswärme durch direkten Wärmetausch abgeführt werden soll, wird der Rückfluß der Kolonne 14 mittels eines zusätzlichen, mit Sattdampf betriebenen Wärmetauschers 26a verdampft. Sofern das in der Kolonne 14 destillierte Gemisch neben 1,2-Dichlorethan noch leichter siedende Komponenten enthält, werden letztere über die Leitung 20 abgezogen und das 1,2-Dichlorethan über die Leitung 25a entnommen. Das Sumpfprodukt der Kolonne 14 wird über die Leitungen 17 und 22a abgezogen und kann einer separaten Aufarbeitung zugeführt werden.

Die Modifizierung des erfindungsgemäßen Verfahrens, bestehend in der Druckminderung in der dem Chlorierungsreaktor nachgeschalteten Fraktionierkolonne, ermöglicht die Überwindung der nachteiligen Folgen unzureichender Druckverhältnisse bei der Addtionschlorierung von Ethylen. So erlaubt die modifizierte Arbeitsweise bei einem vorgegebenen Chlordruck durch Druckminderung in der Fraktionierkolonne die Anteile direkter und indirekter Wärmenutzung zugunsten des direkten Wärmetausches zu verändern, so daß im Extremfall die gesamte nutzbare Reaktionswärme zur Verdampfung des 1,2-Dichlorethans aus dem Reaktionsgemisch herangezogen werden kann, ohne daß dadurch eine Anreicherung von hochsiedenden Nebenprodukten im Reaktionsgemisch bewirkt wird.

Beispiel 1 (Figur 1) :

Es wurde zunächst Reaktor 1, dessen Volumen ca. 25 m³ betrug, mit 20.000 Litern 1,2-Dichlorethan beschickt und die Reaktorfüllung mit 300 mg Eisen-III-chlorid pro kg 1,2-Dichlorethan als Katalysator versetzt. Bei einem Druck von 2,5 bar und einer Temperatur von 105-110 °C wurden in den Reaktor stündlich 2.227 kg Ethylen, 5.737 kg Chlorgas, welches zusätzlich etwa 4 Vol.-% inerte Gase enthielt, sowie 20 Nm³ Luft eingeleitet.

Zur Gewinnung des im Reaktor 1 produzierten 1,2-Dichlorethans sowie der Reaktionswärme wurde aus dem Reaktor 1 ein Flüssigkeitsstrom des Reaktionsgemisches über die Kreislaufleitung 10 entnommen, in zwei Teilströme aufgeteilt, wobei ein Teilstrom seinen Wärmeinhalt an den Wärmeaustauscher 16 abgab und im Kreislauf über die Kreislaufleitung 10 in den Reaktor 1 zurückkehrte, während der zweite Teilstrom über die Leitung 11 dem Entspannungsgefäß 12 zufloß.

Im Wärmetauscher-Kreislauf wurden ca. 350 m³/h Reaktionsgemisch umgepumpt, während aus dem über das Entspannungsgefäß 12 im Kreislauf geführten Teilstrom ca. 8 000 kg rohes 1,2-Dichlorethan verdampfte.

Das über die Leitung 13 der Destillationskolonne 14 zugeführte 1,2-Dichlorethan enthielt etwa 0,1 Gew.-% 1,1,2-Trichlorethan sowie etwa 0,01 Gew.-% Ethylchlorid und 1,1-Dichlorethan. Eine Anreicherung von hochsiedenden Komponenten im Reaktionsgemisch des Reaktors 1 war nicht feststellbar, da die in Nebenreaktionen gebildeten Produkte bei nur 3- bis 4-stündiger Verweilzeit im Reaktionsgemisch mit den Dichlorethan-Dämpfen aus dem Entspannungsgefäß 12 vollständig ausgetragen wurden. Die Sumpftemperatur in der Destillationskolonne 14 betrug ca. 78 °C.

Das Abgas des Reaktors 1 wurde über die Leitung 6 dem Kühler 7 zugeleitet. 102 Nm³/h nicht kondensierte Anteile des Abgases mit einem Gehalt von 2,5 Vol.-% 1,2-Dichlorethan und je 3 Vol.-% Ethylchlorid und Ethylen wurden über die Leitung 8 entnommen und einer Verbrennungsanlage zugeführt.

Über die Leitung 20 der Destillationskolonne 14 wurden die niedrig siedenden Anteile entfernt. Der Sumpf der Kolonne 14 wurde über die Leitungen 17 und 22 zur Kolonne 23 geleitet und dort das 1,1,2-Trichlorethan abgetrennt. Aus dem Seitenabzug 29 der Kolonne 14 konnten 7.972 kg/h 1,2-Dichlorethan entnommen werden.

Beispiel 2 (Figur 2) :

In dem Reaktor 1 wurden bei einem Druck von 3,0 bar und einer Temperatur von 119 °C 5,1 t/h 1,2-Dichlorethan durch Umsetzung von Ethylen mit Chlor in flüssigem 1,2-Dichlorethan in Gegenwart von Eisen-III-chlorid als Chlorierungskatalysator produziert und außerdem zusätzlich 12,4 t/h trockenes Roh-Dichlorethan mit einem Gehalt an hochsiedenden Anteilen von ca. 0,4 Gew.-% und einer Temperatur von ca. 25 °C über die Leitung 23a in die Reaktionszone eingebracht. Vom Reaktor 1 wurden gleichzeitig über die Leitungen 10 und 11 181 t/h Reaktionsgemisch unter Ausnutzung des Druckgefälles dem Entspannungsgefäß 12 zugeführt, in welchem 17,5 t/h Dichlorethan bei einem Druck von etwa 1,4 bar verdampften und über die Leitung 13 der Destillationskolonne 14 zuströmen.

163,5 t/h Reaktionsgemisch kehrten, durch die Kreislaufpumpe 15 gefördert, über die Kreislaufleitungen 11 und 10 bei entsprechend der verdampften Dichlorethanmenge erniedrigter Temperatur in den Reaktor 1 zurück.

In der Kolonne 14 wurden im Sumpf und am Kolonnenkopf Temperaturen von 82° bzw. 68 °C gemessen, wobei der Druck am Kolonnenkopf etwa 0,6 bar und der Differenzdruck zwischen Kopf und Sumpf etwa 0,35 bar betrug. Bei einem Rücklaufverhältnis von etwa 0,6 ergab sich im über die Leitung 25 ablaufenden Rein-Dichlorethan ein Hochsiederanteil von < 0,01 Gew.-%.

Die bei der Reaktion anfallende Reaktionswärme wurde in direktem Wärmetausch vollständig genutzt, so daß die Wärmezufuhr zum Wärmetauscher 16 der Kolonne 14 geschlossen war. Die Verdampfung des Rücklaufes in der Kolonne erfolgte größtenteils mittels eines zusätzlichen, mit Sattdampf betriebenen Wärmetauschers 26a und zum kleineren Teil durch den über Leitung 13 eintretenden überhitzten Dichlorethan-Dampf.

Das Sumpfprodukt der Kolonne 14 mit einem Gehalt von ca. 2,4 Gew.-% an hochsiedenden Anteilen wurde über die Leitung 17 und 22a abgezogen und separat aufgearbeitet.

Beispiel 3 (Vergleichsbeispiel)

Es wurde analog Beispiel 2 verfahren, jedoch wurde die Kolonne 14 bei Normaldruck betrieben. In dem Entspannungsgefäß 12 stellte sich ein Druck von 1,8 bar ein. Im Gegensatz zu Beispiel 2 konnten nicht 17,5 t/h sondern nur noch 12,5 t/h Dichlorethan-Dampf der Destillationskolonne 14 über die Leitung 13 zugeführt werden. Ein Teil der Reaktionswärme mußte über den Wärmetauscher 16 an den Kolonnensumpf abgeführt werden. In den Kolonne 14 betrug die Sumpftemperatur 95 °C und am Kopf der Kolonne wurden 84 °C gemessen.

**Ansprüche**

1. Verfahren zur Herstellung und Reinigung von 1,2-Dichlorethan durch Umsetzung von Ethylen und Chlor in einer Reaktionszone, welche ein umlaufendes flüssiges Medium mit einem Gehalt an chlorierten Kohlenwasserstoffen mit zwei Kohlenstoffatomen enthält, bei einer Temperatur unterhalb der Verdampfungstemperatur des Mediums bei dem in der Reaktionszone herrschenden Druck und in Gegenwart eines üblichen Katalysators zur Chlorübertragung und gegebenenfalls eines Inhibitors zur Verringerung der Nebenproduktbildung, unter Bildung von rohem 1,2-Dichlorethan, welches aus der Reaktionszone abgezogen und in einer anschließenden separaten Fraktionierkolonne gereinigt wird, dadurch gekennzeichnet, daß man

a) etwa äquimolare Mengen von Ethylen und Chlor in das umlaufende flüssige Medium einleitet und nach intensiver Durchmischung in einer Mischzone das Gemisch in einer Reaktionszone bei einer Temperatur von etwa 75-200 °C und einem Druck von etwa 1-15 bar zur Reaktion bringt, wobei die mittlere Verweilzeit des Reaktionsgemisches in der Misch- und Reaktionszone etwa 1-15 Stunden beträgt ;

b) aus der Reaktionszone einen Teil des flüssigen Reaktionsgemisches abzieht und letzteren in zwei Teilströme aufteilt, wobei ein Teilstrom zur Abgabe von Wärmeenergie einen Wärmeaustauscher passiert und danach mit verminderter Temperatur in die Misch- und Reaktionszone zurückfließt, während der zweite Teilstrom einem Entspannungsgefäß zugeführt wird, in welchem eine adäquate Menge des in der Reaktionszone gebildeten Reaktionsproduktes sowie gegebenenfalls ein Anteil nach anderer Verfahrensweise hergestelltes und der Reaktionszone zugeführtes 1,2-Dichlorethan aus dem zweiten Teilstrom verdampft, wobei die Dämpfe in eine Fraktionierkolonne eingeleitet werden, während der nichtverdampfte, flüssige Anteil des zweiten Teilstromes in die Misch- und Reaktionszone des umlaufenden flüssigen Mediums zurückkehrt ;

c) aus den in die Fraktionierkolonne eingeleiteten Dämpfen das 1,2-Dichlorethan destillativ unter Verwendung eines Teiles der im Wärmeaustauscher übertragenen Wärmeenergie abtrennt und letzteres über Kopf der Kolonne abzieht, wobei im Sumpf der Kolonne höherchlorierte Produkte anfallen, die abgezogen und separat aufgearbeitet werden.

2. Verfahren nach Anspruch 1, dadurch gekennzeichnet, daß das umlaufende flüssige Medium überwiegend 1,2-Dichlorethan ist.

3. Verfahren nach Anspruch 1 oder 2, dadurch gekennzeichnet, daß die Umsetzung des Ethylens mit Chlor bei einer Temperatur von 95-160 °C, einem Druck von 1-15 bar und unter Einhaltung einer mittleren Verweilzeit von 2 bis 10 Stunden erfolgt.

4. Verfahren nach Anspruch 1-3, dadurch gekennzeichnet, daß der eine Teilstrom des flüssigen Reaktionsgemisches nach Passieren des Wärmetauschers mit einer um etwa 5 bis 50 °C verminderten Temperatur in die Misch- und Reaktionszone zurückkehrt.

5. Verfahren nach Anspruch 1-4, dadurch gekennzeichnet, daß man im Reaktionsgemisch enthaltene inerte Gase oder leichtsiedende Chlorkohlenwasserstoffe, wie Ethylchlorid, aus dem oberen Teil der Reaktionszone abzieht und in einem nachgeschalteten Kühler kühlt, so daß mit den Abgasen entweichendes Dichlorethan kondensiert, und daß man letzteres in die Reaktionszone zurückleitet oder einem anderen Verwendungszweck zuführt.

6. Verfahren nach Anspruch 1-5, dadurch gekennzeichnet, daß man ein nach anderer Verfahrensweise hergestelltes rohes 1,2-Dichlorethan entweder in den nachgeschalteten Kühler einleitet und über die Abflußleitung des Kühlers der Reaktionszone zuführt, oder daß man es in einen der beiden Teilströme nach Wärmeaustausch bzw. Produktverdampfung vor deren Rückkehr in die Reaktionszone einbringt.

7. Verfahren nach Anspruch 1-6, dadurch gekennzeichnet, daß die aus der Reaktionszone abgezogene Menge umlaufenden Reaktionsgemisches etwa das 3- bis 30-fache des Reaktorvolumens beträgt.

8. Verfahren nach Anspruch 1-7, dadurch gekennzeichnet, daß die anfallende nutzbare Wärmeenergie zur Rektifizierung des gebildeten sowie einer zusätzlichen Menge nach anderer Verfahrensweise hergestellten 1,2-Dichlorethans herangezogen wird.

9. Verfahren nach Anspruch 1-7, dadurch gekennzeichnet, daß die anfallende nutzbare Wärmeenergie ganz oder teilweise außerhalb des Verfahrens für Heizzwecke oder zur Dampferzeugung verwendet wird.

10. Verfahren nach Anspruch 1-9, dadurch gekennzeichnet, daß man an die zur Abtrennung des 1,2-Dichlorethans aus dem Reaktionsgemisch bestimmte Fraktionierkolonne ein Vakuum anlegt derart, daß das Druckverhältnis zwischen Reaktordruck und dem Druck in der Fraktionierkolonne etwa 2 bis 10 zu 1 beträgt.

11. Verfahren nach Anspruch 10, dadurch gekennzeichnet, daß der Druck im Reaktor etwa 1 bis 5 bar und der Druck in der Fraktionierkolonne etwa 0,9 bis 0,2, insbesondere 0,7-0,5 bar beträgt.

**Claims**

1. Process for making and purifying 1,2-dichloroethane by reacting ethylene and chlorine in a reaction zone having a liquid medium containing chlorinated $C_2$-hydrocarbons circulated therein, at a temperature lower than the evaporation temperature of said medium under the pressure prevailing inside the reaction zone, in the presence of a customary chlorination-inducing catalyst and optionally an inhibitor reducing the formation of by-products, with the resultant formation of crude 1,2-dichloroethane, removing the crude dichloroethane from the reaction zone and purifying it in a separate fractionating zone placed downstream thereof, which comprises :

a) introducing approximately equimolar proportions of ethylene and chlorine into the circulated liquid medium ; intensively mixing the whole in a mixing zone and then reacting the mixture in a reaction zone at a temperature of about 75-200 °C under a pressure of about 1-15 bars, the mean sojourn time of the reaction mixture in the mixing and reaction zone being about 1-15 hours ;

b) removing a portion of the liquid reaction mixture from the reaction zone and subdividing said portion into two partial streams, of which one is passed through a heat exchanger to give off calorific energy, and recycled at lower temperature to the mixing and reaction zone ; whilst the second partial stream is introduced into an expansion vessel in which a quantity adequate to the reaction product formed in the reaction zone and optionally also a proportion of 1,2-dichloroethane originating from a third source and introduced into the reaction zone is evaporated from said second partial stream ; resulting vaporous matter is introduced into a fractionating column whilst unevaporated liquid matter of the second partial stream is recycled into the mixing and reaction zone of the circulated liquid medium ; and

c) separating distillatively 1,2-dichloroethane from the vaporous matter introduced into the fractionating column with the use of a portion of the heat energy tranferred inside the heat exchanger and removing the 1,2-dichloroethane overhead, higher chlorinated products being obtained in the column base portion from which they are removed and worked up separately.

2. Process as claimed in claim 1, wherein the liquid medium circulated consists essentially of 1,2-dichloroethane.

3. Process as claimed in claim 1 or 2, wherein the ethylene and chlorine are reacted at a temperature of 95-160 °C, under a pressure of 1-15 bars over a mean period of 2-10 hours.

4. Process as claimed in claims 1 to 3, wherein one of the two partial streams of liquid reaction mixture is passed through the heat exchanger and recycled with a temperature reduced by about 5 to 50 °C to the mixing and reaction zone.

5. Process as claimed in claims 1 to 4, wherein inert gases or low-boiling chlorinated hydrocarbons, such as ethyl chloride, present in the reaction mixture are removed from the upper portion of the reaction zone, cooled in a condenser downstream thereof so as to condense dichloroethane which escapes together with the issuing gas, the condensed dichloroethane being recycled to the reaction zone or used otherwise.

6. Process as claimed in claims 1 to 5, wherein crude 1,2-dichloroethane made by another process is either introduced into the downstream condenser and recycled through the condenser outlet into the reaction zone, or introduced into one of the two partial streams after heat exchange and evaporation of product, respectively, prior to their being recycled to the reaction zone.

7. Process as claimed in claims 1 to 6, wherein the quantity of circulated reaction mixture taken from the reaction zone is about 3-30 times the reactor volume.

8. Process as claimed in claims 1 to 7, wherein resulting utilizable calorific energy is used for rectifying the 1,2-dichloroethane produced and an additional quantity of the 1,2-dichloroethane made by another process.

9. Process as claimed in claims 1 to 7, wherein resulting utilizable calorific energy is wholly or partially utilized outside the process for heating purposes or for the generation of steam.

10. Process as claimed in claims 1 to 9, wherein the fractionating column used for separating 1,2-dichloroethane from the reaction mixture is operated under a vacuum sufficient to establish a ratio between the pressure prevailing in the reactor and that prevailing in the fractionating column of about 2-10 : 1.

11. Process as claimed in claim 10, wherein the reactor is operated under a pressure of about 1-5 bars and the fractionating column is operated under a pressure of about 0.9-0.2 bar, especially 0.7-0.5 bar.

## Revendications

1. Procédé de préparation et de purification de 1,2-dichloroéthane par réaction d'éthylène et de chlore dans une zone de réaction renfermant un milieu liquide circulé qui contient des hydrocarbures en $C_2$ à une température inférieure à la température d'évaporation du milieu sous la pression régnant dans la zone de réaction et en présence d'un catalyseur de transfert de chlore habituel et, éventuellement, d'un inhibiteur diminuant la formation de produits secondaires, avec formation de 1,2-dichloroéthane brut, que l'on soutire de la zone de réaction et que l'on purifie dans une colonne de fractionnement séparée placée à la suite, caractérisé en ce que :

a) on introduit des quantités à peu près équimolaires d'éthylène et de chlore dans le milieu liquide en circulation et, après mélange intensif dans une zone de mélange, on fait réagir le mélange dans une zone de réaction à une température d'environ 75-200 °C et sous une pression d'environ 1-15 bars, la durée de séjour moyenne du mélange réactionnel dans la zone de mélange et de réaction étant d'environ 1-15 h ;

b) on soutire de la zone de réaction une partie du mélange de réaction liquide et on la subdivise en deux courants partiels, dont l'un passe par un échangeur de chaleur pour céder son énergie calorifique et retourne ensuite à température réduite dans la zone de réaction et de mélange, tandis que l'autre courant partiel est envoyé à un récipient de détente, dans lequel on évapore de ce second courant partiel une quantité adéquate du produit de réaction formé dans la zone de réaction ainsi éventuellement qu'une fraction de 1,2-dichloroéthane obtenu par un autre procédé et envoyé à la zone de réaction, les vapeurs étant introduites dans une colonne de fractionnement, tandis que la partie liquide non évaporée du second courant partiel est renvoyée dans la zone de mélange et de réaction du milieu liquide en circulation ;

c) on sépare par distillation le 1,2-dichloroéthane des vapeurs introduites dans la colonne de fractionnement en utilisant une partie de l'énergie calorifique dégagée dans l'échangeur de chaleur et on le soutire en tête de la colonne en obtenant dans le fond de la colonne des produits plus chlorés que l'on soutire et que l'on soumet à un traitement complémentaire séparé.

2. Procédé selon la revendication 1, caractérisé en ce que le milieu liquide en circulation est essentiellement du 1,2-dichloroéthane.

3. Procédé selon la revendication 1 ou 2, caractérisé en ce que l'on effectue la réaction de l'éthylène avec le chlore à une température de 95-160 °C sous une pression de 1-15 bars et en maintenant une durée de séjour moyenne de 2-10 h.

4. Procédé selon les revendications 1 à 3, caractérisé en ce que le courant partiel du mélange de réaction liquide après passage par l'échangeur de chaleur retourne à une température réduite d'environ 5 à 50 °C dans la zone de mélange et de réaction.

5. Procédé selon les revendications 1 à 4, caractérisé en ce que l'on soutire de la partie supérieure de la zone de réaction les gaz inertes ou hydrocarbures de bas point d'ébullition, tel que le chlorure d'éthyle, contenus dans le mélange de réaction et on les refroidit dans un réfrigérant placé à la suite de manière à condenser du dichloroéthane se dégageant avec les gaz résiduaires et on renvoie ce dernier dans la zone de réaction ou on l'envoie à une autre utilisation.

6. Procédé selon les revendications 1 à 5, caractérisé en ce que soit introduit le 1,2-dichloroéthane brut obtenu par un autre procédé dans le réfrigérant placé à la suite et, par la conduite de sortie du réfrigérant, dans la zone de réaction, soit on l'envoie dans l'un des deux courants partiels après échange de chaleur et évaporation du produit, respectivement, avant leur retour dans la zone de réaction.

7. Procédé selon les revendications 1 à 6, caractérisé en ce que la quantité de mélange de réaction en circulation soutirée de la zone de réaction est environ 3 à 30 fois le volume du réacteur.

8. Procédé selon les revendications 1 à 7, caractérisé en ce que l'on utilise l'énergie calorifique utilisable obtenue pour rectifier le 1,2-dichloroéthane formé ainsi qu'une quantité supplémentaire de 1,2-dichloroéthane préparé par un autre procédé.

9. Procédé selon les revendications 1 à 7, caractérisé en ce que l'on utilise l'énergie calorifique utilisable partiellement ou complètement en dehors du procédé à des fins de chauffage ou pour la production de vapeur.

10. Procédé selon les revendications 1 à 9, caractérisé en ce que l'on fait fonctionner la colonne de fractionnement, destinée à la séparation du 1,2-dichloroéthane du mélange de réaction, sous un vide tel que le rapport de pression entre la pression dans le réacteur et la pression dans la colonne de fractionnement soit d'environ 2-10 : 1.

11. Procédé selon la revendication 10, caractérisé en ce que la pression dans le réacteur est d'environ 1-5 bars et la pression dans la colonne de fractionnement est d'environ 0,9-0,2 bar, de préférence 0,7-0,5 bar.

Fig.1

Fig. 2